# EUROPEAN PATENT APPLICATION

(11) **EP 3 143 922 A1**
(43) Date of publication of application: **22.03.2017**
(21) Application number: 15792458.0
(22) Date of filing: 01.05.2015
(51) Int. Cl.: A61B 1/018

(54) **ADAPTER FOR TREATMENT INSTRUMENT, ENDOSCOPE, AND ENDOSCOPE SYSTEM**

(30) Priority: 15.05.2014 JP 2014101387
(71) Applicant: Olympus Corporation, Hachioji-shi Tokyo 192-8507 (JP)
(72) Inventor: ISODA, Takumi, Tokyo 192-8507 (JP)
(74) Representative: Schicker, Silvia
(86) International application number: PCT/JP2015/063127
(87) International publication number: WO 2015/174304

(57) **Abstract**

An object is to move a treatment tool (7) in a wider treatment area in real time, while performing treatment. A treatment-tool adaptor (2) of the present invention includes: an adaptor body (15) that is secured to the tip of an insertion portion (8) of an endoscope; a movable member (16) that is disposed along the longitudinal direction of the insertion portion (8) and that supports the tip portion of a treatment-tool channel member (4) through which a treatment tool (7) is passed; and a moving mechanism (17) that causes the movable member (16) to translationally move relative to the adaptor body (15) in a direction intersecting the longitudinal axis of the treatment-tool channel member (4) supported by the movable member (16).

## Description

### {Technical Field}

The present invention relates to a treatment-tool adaptor, an endoscope, and an endoscope system.

### {Background Art}

There is a known endoscope that has, at the tip of a channel into which a treatment tool provided inside an insertion portion of an endoscope is inserted, a through-hole through which a treatment tool passing through the channel can pass, and that is provided with a guiding base that can turn about an axis perpendicular to the longitudinal axis of the through-hole. With this endoscope, the tip position of the treatment tool is changed by bending the treatment tool via swiveling of the guiding base (for example, see Patent Literature 1).

There is a known cylindrical adaptor that is removably attached to the tip of an insertion portion of an endoscope by means of flexibility (for example, see Patent Literature 2). This adaptor has, at an off-center position, a through-hole through which the treatment tool passes, and the position of the treatment tool relative to the insertion portion is changed by changing the attachment angle with respect to the insertion portion.

### {Citation List}

### {Patent Literature}

{PTL 1} Publication of Japanese Patent No. 4022093
{PTL 2} Publication of Japanese Patent No. 3912726

### {Summary of Invention}

### {Technical Problem}

In the endoscope in Patent Literature 1, the treatment tool itself is made to bend, and therefore, it is difficult to employ it in cases where the treatment tool is highly rigid. In addition, since the treatment tool is moved in a fan shape by bending it, the depth-wise treatment area at the end of the operating area becomes narrow.

With the adaptor in Patent Literature 2, it is necessary to adjust the attachment angle of the adaptor relative to the insertion portion to set the position of the treatment tool, before inserting the insertion portion into the body. Thus, it is difficult to change the tip position of the treatment tool in real time during the treatment, and the system cannot cope with changes in the procedure.

The present invention has been conceived in light of the above circumstances, and provides a treatment-tool adaptor, an endoscope and an endoscope system with which it is possible to move the treatment tool in a wider treatment area in real time, while performing treatment.

### {Solution to Problem}

An aspect of the present invention is a treatment-tool adaptor comprising: an adaptor body that is secured to an insertion portion of an endoscope; a movable member that is disposed along the longitudinal direction of the insertion portion and that supports a treatment-tool channel member through which a treatment tool is passed; and a moving mechanism that causes the movable member to translationally move relative to the adaptor body in a direction intersecting the longitudinal axis of the treatment-tool channel member supported by the movable member.

With this aspect, the treatment-tool channel member is supported in the movable member, and the insertion portion to which the adaptor body is secured is introduced into the body of the patient. The treatment tool introduced into the treatment-tool channel member from the base end of the treatment-tool channel member is made to protrude from an opening in the tip of the treatment-tool channel member, whereby it is possible to dispose the tip portion of the treatment tool within the field of view of the endoscope. Then, by operating the moving mechanism, the treatment-tool channel member supported by the movable member is translationally moved in a direction intersecting the longitudinal axis of the treatment-tool channel member, whereby the treatment tool that passes through the treatment-tool channel member is also made to translationally move together with the treatment-tool channel member. Accordingly, compared with the case where it is moved in a fan shape, it is possible to move the treatment tool in wider treatment area in real time, while performing treatment with the treatment tool.

In this aspect, the moving mechanism may be provided with an elongated hole that accommodates the movable member in a manner allowing movement in the longitudinal direction, elongated members that are secured to the movable member and that flank the movable member and extend to both sides thereof, and elongated-member support portions that support the elongated members in the vicinity of both ends of the elongated hole in such a manner as to allow movement in respective longitudinal directions thereof.

By doing so, when the elongated member extending to one side of the movable member is pulled, the movable member moves in one direction in the longitudinal direction of the elongated hole; therefore, the treatment-tool channel member supported by the movable member translationally moves, and the treatment tool that passes through the treatment-tool channel member can be translationally moved in a simple manner.

In the above-described aspect, the adaptor body may be provided with an insertion-portion securing portion that secures the insertion portion, and the elongated hole may be formed in an arc shape radially outward of the insertion portion secured in the insertion-portion securing portion.

By doing so, the movable member is moved along the arc-shaped elongated hole, whereby the treatment tool can be translationally moved in the circumferential direction along the outer circumferential surface of the insertion portion.

In the above-described aspect, the adaptor body may be provided with an insertion-portion securing portion that secures the insertion portion, and the elongated hole may be formed in a straight-line shape radially outward of the insertion portion secured in the insertion-portion securing portion.

By doing so, the movable member is moved along the straight-line-shaped elongated hole, whereby the treatment tool can be translationally moved in a straight line.

In the above-described aspect, the adaptor body may be provided with an insertion-portion securing portion that secures the insertion portion, and the movable member may be attached in a manner allowing rotation thereof about the axis of the insertion portion secured to the insertion-portion securing portion, and may support the treatment-tool channel member radially outward of the adaptor body.

By doing so, the insertion portion is secured in the insertion-portion securing portion of the adaptor body, and the movable member is rotated about the axis of the insertion portion secured in the insertion-portion securing portion, whereby the treatment-tool channel member supported by the movable member can be translationally moved, radially outward of the adaptor body, in the circumferential direction along the outer circumferential surface of the insertion portion.

In the above-described aspect, a channel securing portion that secures a tip portion of another treatment-tool channel member may be provided in the adaptor body.

By doing so, the other treatment tool can be made to protrude towards the front of the insertion portion via the other treatment-tool channel member secured to the channel securing portion, thus increasing the number of treatment tools and making the system compatible with more complex procedures.

In the above-described aspect, a plurality of the movable members may be provided, and the moving mechanism may be provided in each of the movable members.

By doing so, it is possible to make a plurality of treatment tools protrude towards the front of the insertion portion via the plurality of treatment-tool channel members supported in the plurality of movable members, and by moving the plurality of treatment tools individually, it is possible to increase the number of treatment tools, thus making the system compatible with more complex procedures.

Another aspect of the present invention is an endoscope comprising: an insertion portion; a treatment-tool channel member that is disposed along the longitudinal direction inside the insertion portion and through which a treatment tool passes; a movable member that supports the treatment-tool channel member; and a moving mechanism that causes the movable member to translationally move relative to the insertion portion in a direction intersecting the longitudinal axis of the treatment-tool channel member supported by the movable member.

Another aspect of the present invention is an endoscope system comprising: an endoscope; any one of the above-described treatment-tool adaptors, which is attached to the tip of an insertion portion of the endoscope; a treatment-tool channel member that is secured to the movable member of the treatment-tool adaptor; and a treatment tool that passes through the treatment-tool channel member and the tip portion of which is made to protrude at the tip of the insertion portion.

### {Advantageous Effects of Invention}

The present invention affords an advantage in that it is possible to move a treatment tool in a wider treatment area in real time, while performing treatment.

### {Brief Description of Drawings}

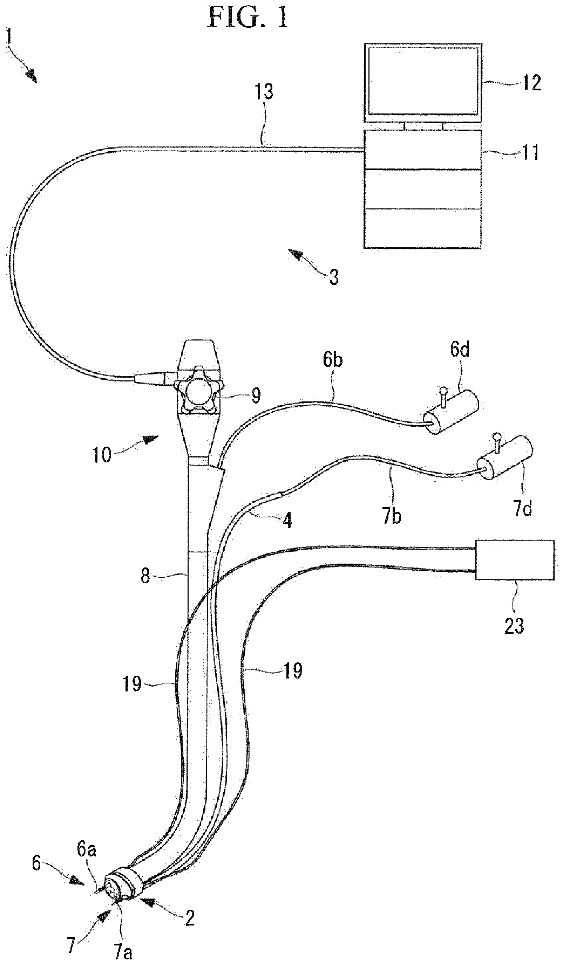

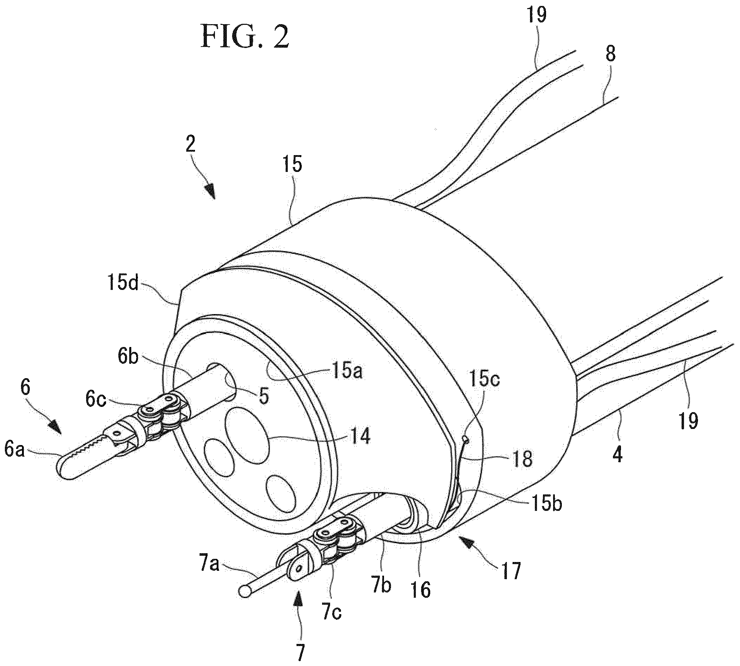

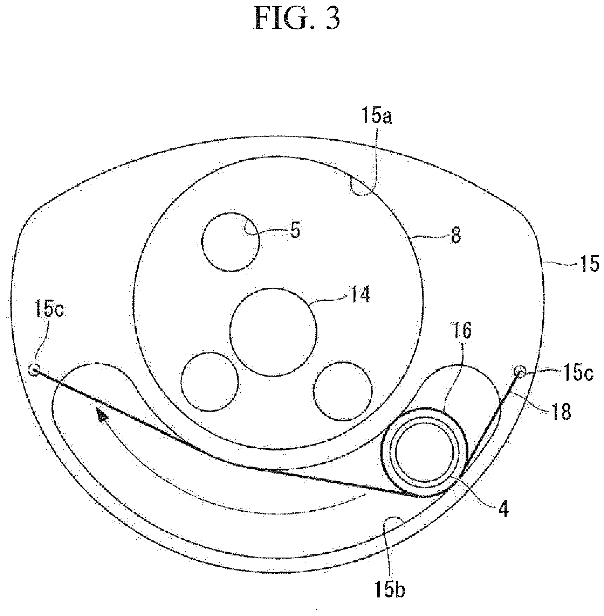

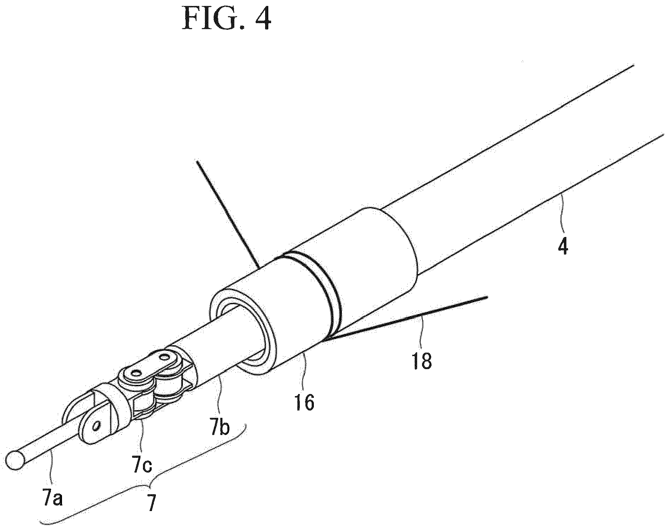

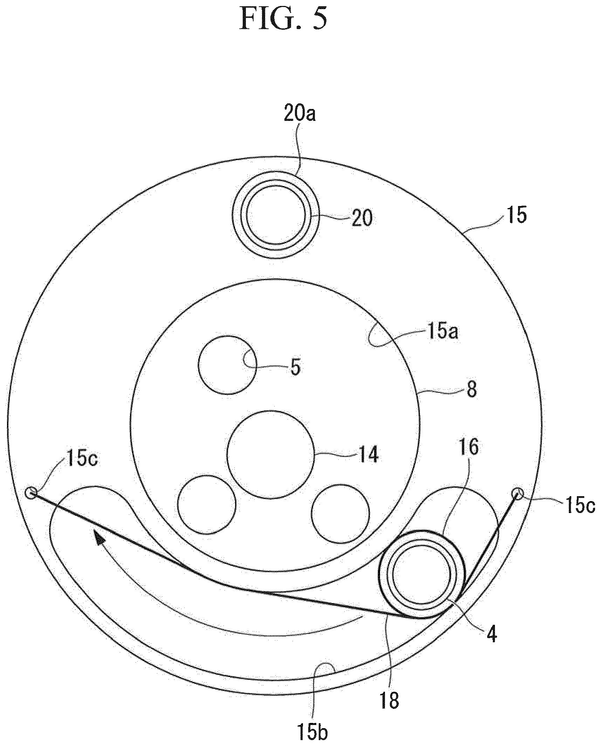

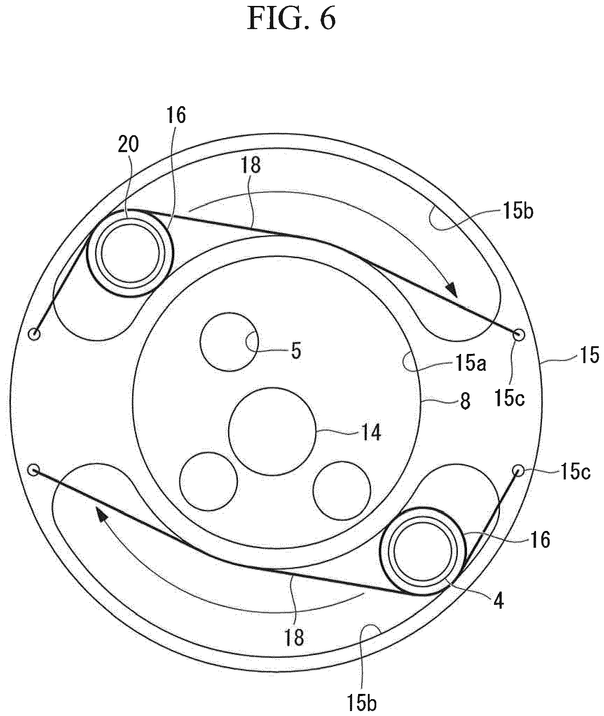

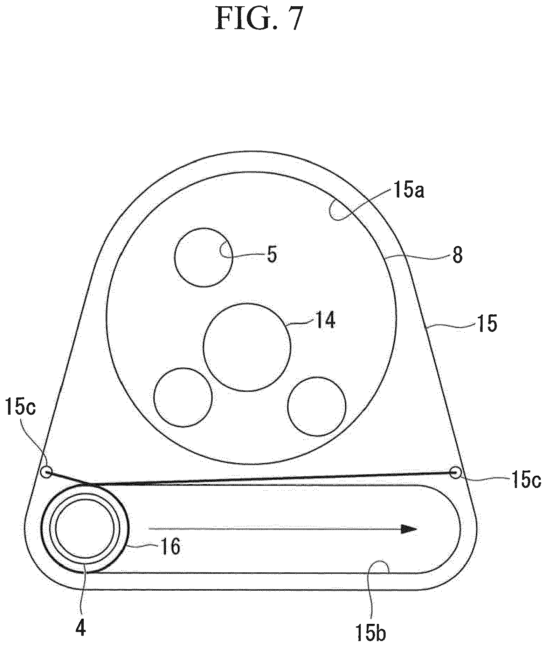

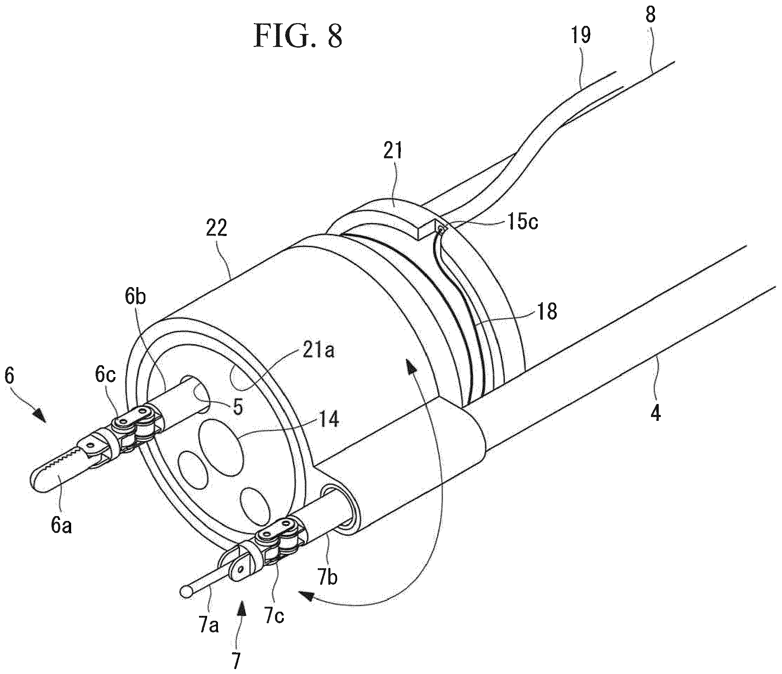

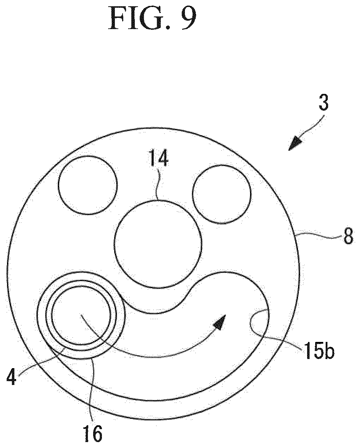

{Fig. 1} Fig. 1 is diagram showing the overall configuration of an endoscope system according to an embodiment of the present invention.
{Fig. 2} Fig. 2 is a perspective view showing a treatment-tool adaptor according to an embodiment of the present invention, provided in the endoscope system in Fig. 1.
{Fig. 3} Fig. 3 is a front view showing the treatment-tool adaptor in Fig. 2.
{Fig. 4} Fig. 4 is a perspective view showing a tip portion of the treatment tool provided in the endoscope system in Fig. 1.
{Fig. 5} Fig. 5 is a front view showing a first modification of the treatment-tool adaptor in Fig. 2.
{Fig. 6} Fig. 6 is a front view showing a second modification of the treatment-tool adaptor in Fig. 2.
{Fig. 7} Fig. 7 is a front view showing a third modification of the treatment-tool adaptor in Fig. 2.
{Fig. 8} Fig. 8 is a perspective view showing a fourth modification of the treatment-tool adaptor in Fig. 2.
{Fig. 9} Fig. 9 is a front view showing an endoscope according to an embodiment of the present invention.

### {Description of Embodiments}

An endoscope system 1 and a treatment-tool adaptor 2 according to an embodiment of the present invention will be described below with reference to the drawings.

As shown in Fig. 1, the endoscope system 1 according to this embodiment includes an endoscope 3, the treatment-tool adaptor 2, which is combined with the endoscope, a treatment-tool channel member 4 that is attached to the treatment-tool adaptor 2, and two treatment tools 6 and 7 that are respectively introduced via channels 5 provided in the treatment-tool channel member 4 and the endoscope 3.

The endoscope 3 includes: an endoscope body 10 provided with a long, thin, flexible insertion portion 8 that is inserted into the body of a patient and a manipulation unit 9 for manipulating a bending portion at the tip of the insertion portion 8; a light source and image processing unit 11; and a monitor 12. In the figure, reference sign 13 is an optical fiber that connects the light source and image processing unit 11 with the endoscope body 10 and that guides light from the light source, as well as a cable that transmits electrical signals between the endoscope body 10 and the image processing unit 11.

The image processing unit 11 processes image signals obtained by an optical system 14 disposed at the tip of the insertion portion 8. The image processing unit 11 is, for example, a computer. The monitor 12 displays images processed by the image processing unit 11.

As shown in Fig. 1 and Fig. 2, the treatment-tool adaptor 2 according to this embodiment includes an adaptor body 15 that is secured to the tip of the insertion portion 8 in the endoscope 3, a movable member 16 that is movable supported in the adaptor body 15, and a moving mechanism 17 that moves the movable member 16.

The adaptor body 15 includes a through-hole (insertion-portion securing portion) 15 into which the insertion portion 8 is fitted; an elongated hole 15b that has an axis parallel to the longitudinal axis of the through-hole 15a and that is open over a prescribed angular range radially outward of the through-hole 15a; and wire support holes 15c (elongated-member support portions) through which wires (described later) are made to pass. Reference sign 15d in the figure is a tapered portion that narrows toward the tip of the adaptor body 15 for improving the insertability of the insertion portion 8, to which the adaptor body 15 is fitted, into the body.

As shown in Fig. 3 and Fig. 4, the movable member 16 is a cylindrical member that is secured in a state in which the tip of the treatment-tool channel member 4 is fitted therein. The outer diameter dimension of the movable member 16 is formed to be slightly smaller than the inner diameter dimension of the elongated hole 15b. Accordingly, the movable member 16 can move in the longitudinal direction of the elongated hole 15b, inside the elongated hole 15b, while the longitudinal axis of the treatment-tool channel member 4 is kept parallel to the longitudinal axis of the insertion portion 8.

As shown in Figs. 1 to 4, the moving mechanism 17 includes: the wires (elongated members) 18 that are wound on the outer circumferential surface of the movable member 16; the elongated hole 15b; the two wire-support holes 15c, which are provided in the vicinity of both ends of the elongated hole 15b; sheaths 19, which are secured to the base end of the adaptor body 15 and from which the wires 18 passing through inside the wire support holes 15c are led out to the base end of the insertion portion 8; and a wire driving apparatus 23 that gives pulling forces to the wires 18, at the base end of the sheaths 19. The wire driving apparatus 23 is driven so as to pull either one of the two wires 18 led out from the adaptor body 15 and lets out the other. For example, this can be realized by a pulley (not illustrated), a cylinder etc. (not illustrated) on which the wires 18 are wound.

The wires 18, which are guided to the tip of the insertion portion 8 via the sheaths 19, pass via the two wires support holes 15c and are wound on the movable member 16 between the two wire support holes 15c. By doing so, when pulling forces are applied to the wires 18 to pull them, the movable member 16 is pulled towards the wire support holes 15c provided at the ends of the elongated hole 15b, so that it is moved along the elongated hole 15b.

The treatment-tool channel member 4 is a soft tube-shaped member.

In the example shown in Fig. 2, the treatment tools 6 and 7 include end effectors 6a and 7a formed of gripping forceps or energy forceps disposed at the tips thereof; insertion portions 6b and 7b disposed so as to pass through the treatment-tool channel member 4 and the channel 5; joints 6c and 7c that are disposed at the tips of the insertion portions 6b and 7b and that change the angles of the end effectors 6a and 7a; and manipulators 6d and 7d that are disposed at the base ends of the insertion portions 6b and 7b and with which inputs are performed for driving the joints 6c and 7c and for operating the end effectors 6a and 7a.

The operation of the thus-configured endoscope system 1 and treatment-tool adaptor 2 according to this embodiment will be described below.

To perform treatment of an affected area inside the body of a patient by using the endoscope system 1 according to this embodiment, the tip of the insertion portion 8 of the endoscope 3 is fitted into the through-hole 15a in the adaptor body 15 in the treatment-tool adaptor 2 according to this embodiment and is secured. Also, the treatment-tool channel member 4 is fitted in the movable member 16 provided in the treatment-tool adaptor 2.

Then, by emitting illumination light from the light source and operating the endoscope 3, an image of inside the body is obtained by the optical system 14 at the tip of the insertion portion 8 and, after being subjected to image processing by the image processing unit 11, is displayed on the monitor 12. Accordingly, the operator can search for an affected area while looking at the monitor 12, and when an affected area is found, the distal end face of the insertion portion 8 is made to face the affected area that is found.

In the state in which the distal end face of the insertion portion 8 faces the affected area, the treatment tools 6 and 7 are inserted into the channel 5 provided in the insertion portion 8 and the treatment-tool channel member 4 attached to the movable member 16, and as shown in Fig. 2, the end effectors 6a and 7a at the tips are made to project towards the front of the insertion portion 8. By manipulating the manipulators 6d and 7d at the base ends of the insertion portions 6b and 7b, treatment is performed while driving the joints 6c and 7c of the treatment tools 6 and 7 to adjust the angles of the end effectors 6a and 7a.

In this case, in the state in which tissue is gripped by the end effector 6a, which is formed of gripping forceps, if it is desired to move the end effector 7a, which is formed of energy forceps, the wire driving apparatus 23 is operated to pull either one of the wires 18 that extend to both ends of the movable member 16. Accordingly, since the movable member 16 moves along the elongated hole 16b, the opening at the tip of the treatment-tool channel member 4, which is supported in the movable member 16, moves along the elongated hole 16b, and the treatment tool 7, which is inserted into the treatment-tool channel member 4, also translationally moves while maintaining the orientation thereof.

In this case, with the endoscope system 1 and the treatment-tool adaptor 2 according to this embodiment, since the treatment tool 7 translationally moves rather than being displaced in a fan shape, as in the related art, an advantage is afforded in that it is possible to ensure a wide operating area covering the entire operating area of the treatment tool 7. In addition, an advantage is also afforded in that, with the treatment tool 7 remaining inserted inside the body of the patient, the position thereof can be moved in real time.

In this embodiment, as shown in Fig. 5, a channel securing portion 20a that secures another treatment-tool channel member 20 for allowing another treatment tool (not illustrated) to pass therethrough may be provided in the adaptor body 15. Accordingly, the number of treatment tools can be increased, making the system compatible with more complex procedures.

Although the through-hole 15a in which the insertion portion 8 is fitted is illustrated as an example of the insertion-portion securing portion for securing the insertion portion 8 to the adaptor body 15, the insertion-portion securing portion may have another shape, for example, a groove shape, so long as it has a structure that allows the insertion portion to be secured in a positionally aligned manner.

Instead of securing the other treatment-tool channel member 20 to the adaptor body 15, as shown in Fig. 6, a plurality of treatment-tool channel members 20 may be supported in a manner enabling them to move along the elongated hole 15b.

It has been assumed in this embodiment that the treatment-tool channel members 4 and 20 are displaced along arc-shaped elongated holes 15b. Accordingly, the amount of protrusion in the radial direction of the adaptor body 15 is minimized; instead of this, however, as shown in Fig. 7, the elongated hole 15b may have a straight line shape.

Although the movable member 16 that moves inside the elongated hole 15b has been illustrated as an example, instead of this, as shown in Fig. 8, a cylindrical movable member 22 may be attached to the outer face of an adaptor body 21 having a through-hole 21a into which the tip of the insertion portion 8 is fitted, in a manner allowing the cylindrical movable member 22 to rotate about the central axis of the through-hole 21a.

A wire 18, which is wound on the adaptor body 21 via a wire support hole 15c provided in the adaptor body 21, is secured to the movable member 22, whereby when the wire 18 is pulled, the movable member 22 is made to rotate relative to the adaptor body 21. Accordingly, it is possible to translationally move the treatment tool sheath member 4 fixed to the movable member 22 so as to rotate about the through-hole 21a.

In the present invention, it has been assumed that the treatment-tool adaptor 2 is attached to the tip of the insertion portion 8; instead of this, however, as shown in Fig. 9, the endoscope 3 may be configured such that the elongated hole 15b is provided in the distal end face of the insertion portion 8, and the treatment-tool channel member 4, which passes through the interior of the insertion portion 8, is moved along the elongated hole 15b.

By doing so, the treatment-tool adaptor 2 is not attached, and therefore, an advantage is afforded in that it is possible to make the tip of the insertion portion 8 narrower.

It is preferable to support the tip portion of the treatment-tool channel member 4 in the movable member 16; however, the location at which it is supported is not restricted to the tip portion; for example, the treatment-tool channel member 4 may be supported in the movable member 16 at the root (base side) thereof. In this case, the treatment-tool channel member 4 preferably has a certain rigidity.

As in the above-described embodiment, it is preferable to secure the adaptor body 15 to the tip of the insertion portion 8 in the endoscope 3. In this embodiment, the adaptor body 15 has a shape that covers only the tip portion of the insertion portion 8 in the endoscope 3; however, it is not limited to the tip and may have an outer tube shape that covers the entire insertion portion 8 in the endoscope 3.

In this embodiment, the wires 18 have been illustrated as examples of the elongated members; however, they are not limited thereto. Any other elongated members may be employed, so long as they can be secured to the outer circumferential surface of the movable member 16 and can transmit the pulling forces.

In this embodiment, as the elongated hole 15b, one in which the movable member 16 translationally moves while keeping the longitudinal axis of the inserted movable member 16 and the treatment tool 7 parallel to the longitudinal axis of the insertion portion 8 has been illustrated as an example; however, it is not limited thereto. For example, one in which the movable member 16 translationally moves while keeping the longitudinal axis of the movable member 16 and the treatment tool 7 inclined at a fixed angle with respect to the longitudinal axis of the insertion portion 8 may be employed.

By doing so, it is possible to translationally move the movable member 16 while keeping the longitudinal axis of the treatment tool 7 always inclined in a direction towards the center of the field of view of the endoscope.

### {Reference Signs List}

- 1: endoscope system
- 2: treatment-tool adaptor
- 3: endoscope
- 4: treatment-tool channel member
- 6, 7: treatment tool
- 8: insertion portion
- 15: adaptor body
- 15a: through-hole (insertion-portion securing portion)
- 15b: elongated hole
- 15c: wire support hole (elongated-member support portion)
- 16, 22: movable member
- 17: moving mechanism
- 18: wire (elongated member)
- 20a: channel securing portion

## Claims

1. A treatment-tool adaptor comprising:
an adaptor body that is secured to an insertion portion of an endoscope;
a movable member that is disposed along the longitudinal direction of the insertion portion and that supports a treatment-tool channel member through which a treatment tool is passed; and
a moving mechanism that causes the movable member to translationally move relative to the adaptor body in a direction intersecting the longitudinal axis of the treatment-tool channel member supported by the movable member.

2. A treatment-tool adaptor according to claim 1, wherein the moving mechanism is provided with an elongated hole that accommodates the movable member in a manner allowing movement in the longitudinal direction, elongated members that are secured to the movable member and that flank the movable member and extend to both sides thereof, and elongated-member support portions that support the elongated members in the vicinity of both ends of the elongated hole in such a manner as to allow movement in respective longitudinal directions thereof.

3. A treatment-tool adaptor according to claim 2, wherein the adaptor body is provided with an insertion-portion securing portion that secures the insertion portion, and the elongated hole is formed in an arc shape radially outward of the insertion portion secured in the insertion-portion securing portion.

4. A treatment-tool adaptor according to claim 2, wherein the adaptor body is provided with an insertion-portion securing portion that secures the insertion portion, and the elongated hole is formed in a straight-line shape radially outward of the insertion portion secured in the insertion-portion securing portion.

5. A treatment-tool adaptor according to claim 1, wherein the adaptor body is provided with an insertion-portion securing portion that secures the insertion portion, and the movable member is attached in a manner allowing rotation thereof about the axis of the insertion portion secured to the insertion-portion securing portion, and supports the treatment-tool channel member radially outward of the adaptor body.

6. A treatment-tool adaptor according to one of claims 1 to 4, wherein a channel securing portion that secures a tip portion of another treatment-tool channel member is provided in the adaptor body.

7. A treatment-tool adaptor according to one of claims 1 to 6, wherein a plurality of the movable members are provided, and the moving mechanism is provided in each of the movable members.

8. An endoscope comprising:
an insertion portion;
a treatment-tool channel member that is disposed along the longitudinal direction inside the insertion portion and through which a treatment tool passes;
a movable member that supports the treatment-tool channel member; and
a moving mechanism that causes the movable member to translationally move relative to the insertion portion in a direction intersecting the longitudinal axis of the treatment-tool channel member supported by the movable member.

9. An endoscope system comprising:
an endoscope;
a treatment-tool adaptor according to one of claims 1 to 7, which is attached to the tip of an insertion portion of the endoscope;
a treatment-tool channel member that is secured to the movable member of the treatment-tool adaptor; and
a treatment tool that passes through the treatment-tool channel member and the tip portion of which is made to protrude at the tip of the insertion portion.
